(19) **Europäisches Patentamt / European Patent Office / Office européen des brevets**

(11) **EP 4 726 031 A1**

(12) **EUROPEAN PATENT APPLICATION**
published in accordance with Art. 153(4) EPC

(43) Date of publication:
**15.04.2026  Bulletin 2026/16**

(21) Application number: **24818207.3**

(22) Date of filing: **04.01.2024**

(51) International Patent Classification (IPC):
**C12N 5/10** (2006.01)          **C07K 19/00** (2006.01)
**C12N 15/62** (2006.01)          **A61K 39/00** (2006.01)
**A61P 35/02** (2006.01)

(86) International application number:
**PCT/CN2024/070562**

(87) International publication number:
**WO 2024/250668 (12.12.2024 Gazette 2024/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.06.2023  CN 202310683451**

(71) Applicant: **Carbiogene Therapeutics Co., Ltd.
Hangzhou, Zhejiang 310051 (CN)**

(72) Inventors:
• **YANG, Xin
Hangzhou, Zhejiang 310051 (CN)**

• **ZHU, Jiangao
Hangzhou, Zhejiang 310051 (CN)**
• **YANG, Wenjun
Hangzhou, Zhejiang 310051 (CN)**
• **YU, Jiandong
Hangzhou, Zhejiang 310051 (CN)**

(74) Representative: **Lorenz Seidler Gossel Part. mbB
Widenmayerstr. 23
80538 München (DE)**

Remarks:
The complete document including Reference
Table(s) and the Sequence Listing(s) can be
downloaded from the EPO website

(54) **CLL1-CAR-T CELL, AND PREPARATION METHOD THEREFOR AND USE THEREOF**

(57)      This disclosure discloses a CLL1-CAR-T cell, as well as their preparation methods and applications. Specifically, it reveals CLL1-CAR-T cell that contain a chimeric antigen receptor, which comprises a single-domain antibody, a hinge region, a transmembrane region, and an intracellular signaling region. The amino acid sequence of the single-domain antibody corresponds to positions 22-150 of SEQ ID No.1. The CLL1-VHH-1 CAR-T cells of this disclosure can effectively secrete the T-cell-specific effector molecule IFN-$\gamma$, specifically and efficiently kill CLL1$^+$ target cells, and exhibit favorable in vivo anti-tumor activity. They not only significantly inhibit the proliferation of tumor cells in mice but also markedly prolong the survival time of mice. The CLL1-VHH-1 CAR-T cells of this disclosure demonstrate excellent anti-tumor capabilities and can be used for immunotherapy of diseases related to the CLL1 target, such as acute myeloid leukemia, presenting broad prospects for clinical applications.

**EP 4 726 031 A1**

**Description**

**TECHNICAL FIELD**

The present disclosure belongs to the technical field of cellular immunotherapy, specifically relating to a CLL1-CAR-T cell, a method for preparing same and use thereof.

**BACKGROUND**

Acute Myeloid Leukemia (AML) is a malignant disease of myeloid hematopoietic stem/progenitor cells, primarily the abnormal proliferation of primitive and immature myeloid cells in the bone marrow and peripheral blood. Clinically, it manifests as anemia, bleeding, infection and fever, organ infiltration, metabolic abnormalities, etc. Most cases are acute and severe with a poor prognosis, often posing a life-threatening risk if not treated promptly. For a long time, anthracyclines and cytarabine chemotherapy have been used to treat mild patients, while hematopoietic stem cell transplantation has been employed for intermediate- and high-risk patients. The heterogeneity of acute myeloid leukemia makes the current treatment of AML patients still a challenge. In recent years, researchers have discovered that immune-targeted therapy targeting CLL1 has shown remarkable efficacy in treating AML. C-type lectin-like molecule 1 (CLL1), also known as C-type lectin domain family 12 member A (CLEC12A), is a type II transmembrane glycoprotein that plays a crucial role in immune regulation as an inhibitory receptor. CLL1 is present in myeloid cells of the peripheral blood and bone marrow, as well as in most AML cells. It is also expressed on the $CD34^+CD38^-$ stem cells of the majority of AML cases, whereas it is not expressed on the $CD34^+CD38^-$ stem cells of healthy individuals. Due to its unique expression pattern, CLL1 has emerged as a potential target for the treatment and diagnosis of AML. Additionally, CLL1 is expressed on cells of Myelodysplastic Syndromes (MDS) and Chronic Myeloid Leukemia (CML, also known as chronic granulocytic leukemia).

Traditional radiotherapy, chemotherapy, and hematopoietic stem cell transplantation have shown low efficacy in treating AML. However, the advent of CAR-T cell therapy has made it possible to cure AML. CAR-T, short for Chimeric Antigen Receptor T-Cell Immunotherapy, is a form of immunotherapy that modifies immune cells using genetic engineering techniques to express exogenous anti-tumor genes (CAR genes). This modification enables immune cells, such as lymphocytes, to recognize tumor cell surface antigens and specifically target and kill tumor cells without the restriction of major histocompatibility complex (MHC). The structure of CAR mainly consists of an extracellular domain that recognizes tumor cell surface antigens and an intracellular signal transduction domain. The extracellular domain is used to specifically recognize tumor surface-specific proteins (antigens), while the intracellular domain contains co-stimulatory molecule domains that initiate an immune response in lymphocytes upon recognition of tumor surface-specific proteins (antigens), exerting cytotoxic effects on specific target cells (tumor cells) and thereby killing them. Currently, CAR-T cell therapy has achieved encouraging results in treating hematological malignancies such as B-cell tumors. However, the antigenic properties of myeloid cells differ fundamentally from those of B-cell lineage. The killing of normal myeloid cells by CAR-T can lead to bone marrow failure and a lack of corresponding alternative treatments, which has significantly slowed the progress of CAR-T therapy for AML. There are still numerous challenges in successfully applying CAR-T technology to acute myeloid leukemia (AML). Therefore, in-depth research and development of novel and effective CAR-T cells, as well as improving the efficacy of CAR-T cells, hold significant theoretical importance and clinical application value for the immune cell therapy of AML.

**SUMMARY**

One of the objectives of the present disclosure is to provide a CLL1-targeted CAR-T cell (CLL1-CAR-T cell) for the treatment of Acute Myeloid Leukemia (AML). The technical problems to be addressed are not limited to the described technical subject matter, and other technical subject matters not mentioned herein can be clearly understood by those skilled in the art through the following description.

In order to achieve the aforementioned objective, the present disclosure first provides a CLL1-CAR-T cell, which comprises a Chimeric Antigen Receptor (CAR). The chimeric antigen receptor may comprise a single-domain antibody, a hinge region, a transmembrane region, and an intracellular signaling region. The amino acid sequence of the single-domain antibody may be positions 22-150 of SEQ ID No.1.

The CLL1-CAR-T cells (i.e., T cells expressing CLL1-CAR or T cells modified with CLL1-CAR) may be CAR-T cells targeting CLL1 (C-type lectin-like molecule 1). These CLL1-CAR-T cells express the aforementioned chimeric antigen receptor.

Furthermore, the intracellular signaling region may comprise the co-stimulatory domain 4-1BB and the activation domain CD3ζ. The amino acid sequence of 4-1BB may be positions 220-266 of SEQ ID No.1, and the amino acid sequence of CD3ζ may be positions 267-378 of SEQ ID No.1.

Moreover, the hinge region may be a CD8a hinge region with an amino acid sequence of positions 151-195 of SEQ ID No.1, and the transmembrane region may be a CD8a transmembrane region with an amino acid sequence of positions 196-219 of SEQ ID No.1.

Additionally, the CLL1-CAR-T cells may further comprise a signal peptide, whose amino acid sequence may be positions 1-21 of SEQ ID No.1.

Furthermore, the chimeric antigen receptor may be named CLL1-VHH-1 CAR and may comprise any of the following:

A1) a protein with the amino acid sequence of SEQ ID No.1 or positions 22-378 of SEQ ID No.1;

A2) a protein with more than 80% identity to the protein shown in A1) and having the same function, obtained by substitution, deletion, and/or addition of amino acid residues in the amino acid sequence shown in SEQ ID No.1 or positions 22-378 of SEQ ID No.1; and

A3) a fusion protein with the same function as A1), obtained by attaching a tag or signal peptide to the N-terminus and/or C-terminus of A1) or A2).

Furthermore, the attachment in A3) is achieved through peptide bonds.

The tags mentioned herein comprise, but are not limited to: GST (glutathione S-transferase) tag protein, His tag protein (His-tag), MBP (maltose-binding protein) tag protein, Flag tag protein, SUMO tag protein, HA tag protein, Myc tag protein, GFP (green fluorescent protein), CFP (cyan fluorescent protein), YFP (yellow-green fluorescent protein), mCherry (monomeric red fluorescent protein), or AviTag tag protein.

Those skilled in the art can readily mutate the nucleotide sequence encoding the chimeric antigen receptor of the present disclosure using known methods, such as directed evolution or point mutation techniques. Nucleotides that have been artificially modified and exhibit 75% or higher identity to the nucleotide sequence of the chimeric antigen receptor described in the present disclosure, provided that they encode the chimeric antigen receptor and possess its function, are considered to be derived from and equivalent to the nucleotide sequence of the present disclosure.

Any chimeric antigen receptor described herein also falls within the scope of protection of the present disclosure.

The chimeric antigen receptor may sequentially comprise, from the N-terminus to the C-terminus, the signal peptide, the single-domain antibody, the hinge region, the transmembrane region, the co-stimulatory domain, and the activation domain.

The present disclosure also provides biological materials, which may be any one of the following:

B1) a nucleic acid molecule encoding any of the chimeric antigen receptors described herein;

B2) an expression cassette containing the nucleic acid molecule in B1);

B3) a recombinant vector containing the nucleic acid molecule described in B1), or a recombinant vector containing the expression cassette in B2); and

B4) a recombinant microorganism containing the nucleic acid molecule described in B1), or a recombinant microorganism containing the expression cassette in B2), or a recombinant microorganism containing the recombinant vector in B3).

Among them, the expression cassette described in B2), the recombinant vector described in B3), and the recombinant microorganism described in B4) are all capable of expressing the nucleic acid molecule encoding any of the chimeric antigen receptors described herein.

Among the aforementioned biological materials, the nucleic acid molecule described in B1) may be any one of the following:

C1) a DNA molecule whose nucleotide sequence or coding sequence is represented by positions 64-1134 of SEQ ID No.2;

C2) a DNA molecule with 75% or higher identity to the nucleotide sequence defined in C1) and having the same function;

C3) a DNA molecule whose nucleotide sequence or coding sequence is represented by SEQ ID No.2;

C4) a DNA molecule with 75% or higher identity to the nucleotide sequence defined in C3) and having the same function.

The DNA molecule represented by SEQ ID No.2 may be named as the CLL1-VHH-1 CAR gene, which encodes the chimeric antigen receptor CLL1-VHH-1 CAR with an amino acid sequence represented by SEQ ID No.1.

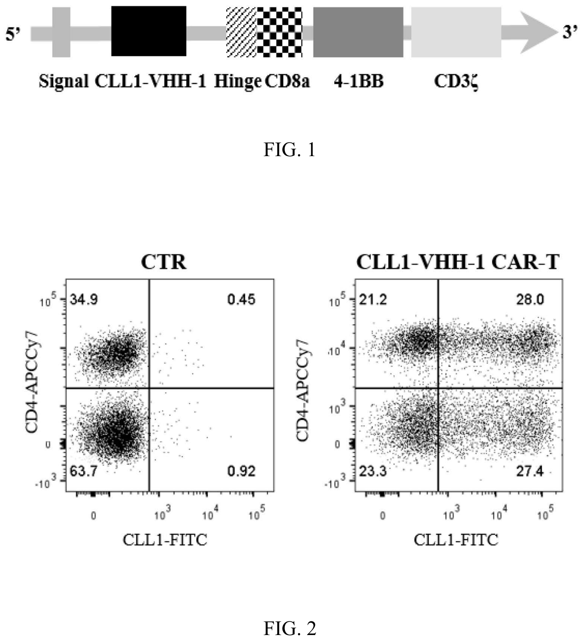

In one embodiment of the present disclosure, the chimeric antigen receptor is named CLL1-VHH-1 CAR. From the N-terminus to the C-terminus, it sequentially comprises a signal peptide (Signal), a single-domain antibody (CLL1-VHH-1), a CD8a hinge region (Hinge), a CD8a transmembrane region, a co-stimulatory domain 4-1BB, and an activation domain CD3ζ (FIG. 1). Among them, the single-domain antibody CLL1-VHH-1 is a single-domain antibody that specifically binds to the CLL1 protein. It serves as the extracellular antigen-binding region of the CAR, responsible for recognizing and binding to the tumor surface antigen CLL1. The N-terminus of the antigen-binding region is equipped with a signal peptide (Signal) that guides the peptide chain of the antigen-binding region to the extracellular space, thereby recognizing the tumor cell surface antigen CLL1. The CD8a hinge region (Hinge) is a peptide chain of the CAR located extracellularly, connecting the extracellular antigen-binding region and the CD8a transmembrane region. The CD8a transmembrane region anchors the single-domain antibody CLL1-VHH-1 to the cell membrane. The intracellular signaling region comprises the co-stimulatory domain 4-1BB and the activation domain CD3ζ, which work together to stimulate T cells, activate intracellular signals, enable T cells to continuously proliferate and release cytokines, enhance the anti-tumor ability of T cells, and simultaneously exert T cell signal transduction functions, promoting a more robust and sustained T cell response.

The amino acid sequence of CLL1-VHH-1 CAR is shown in SEQ ID No.1 (378 aa). Positions 1-21 of SEQ ID No.1 represent the amino acid sequence of the signal peptide (Signal); positions 22-150 represent the amino acid sequence of the single-domain antibody CLL1-VHH-1; positions 151-195 represent the amino acid sequence of the CD8a hinge region (Hinge); positions 196-219 represent the amino acid sequence of the CD8a transmembrane region; positions 220-266 represent the amino acid sequence of the co-stimulatory domain 4-1BB; and positions 267-378 represent the amino acid sequence of the activation domain CD3ζ.

The encoding gene for CLL1-VHH-1 CAR is the CLL1-VHH-1 CAR gene, with its nucleotide sequence shown in SEQ ID No.2 (1134 bp). Positions 1-63 of SEQ ID No.2 represent the nucleotide sequence of the signal peptide (Signal); positions 64-450 represent the nucleotide sequence of the single-domain antibody CLL1-VHH-1; positions 451-585 represent the nucleotide sequence of the CD8a hinge region (Hinge); positions 586-657 represent the nucleotide sequence of the CD8a transmembrane region; positions 658-798 represent the nucleotide sequence of the co-stimulatory domain 4-1BB; and positions 799-1134 represent the nucleotide sequence of the activation domain CD3ζ.

The aforementioned identity of 75% or higher may comprise identities of 80%, 85%, 90%, or 95% or higher.

An identity of over 80% may refer to at least 80%, 81%, 82%, 83%, 84%, 85%, 86%, 87%, 88%, 89%, 90%, 91%, 92%, 93%, 94%, 95%, 96%, 97%, 98%, or 99%. An identity of over 85% may refer to at least 85%, 86%, 87%, 88%, 89%, 90%, 91%, 92%, 93%, 94%, 95%, 96%, 97%, 98%, or 99%. An identity of over 90% may refer to at least 90%, 91%, 92%, 93%, 94%, 95%, 96%, 97%, 98%, or 99%. An identity of over 95% may refer to at least 95%, 96%, 97%, 98%, or 99%.

In this application, identity refers to the identity of amino acid sequences or nucleotide sequences. The identity of amino acid sequences can be determined using homology search websites on the internet, such as the BLAST webpage on the NCBI homepage. For example, in Advanced BLAST 2.1, by using blastp as the program, setting the Expect value to 10, turning off all Filters, using BLOSUM62 as the Matrix, and setting the Gap existence cost, the Per residue gap cost, and the Lambda ratio to 11, 1, and 0.85 (default values), respectively, and performing a search, the identity of amino acid sequences can be calculated, and then the identity value (%) can be obtained.

The vectors mentioned herein refer to those capable of transporting exogenous DNA or target genes into host cells for amplification and expression. The vectors can be cloning vectors or expression vectors, including but not limited to plasmids, phages (such as λ phage or M13 filamentous phage), cosmids (i.e., cosmid vectors), viral vectors (such as baculovirus vectors, retroviruses (including lentiviruses), adenoviruses, adeno-associated viruses, or herpesviruses (such as herpes simplex virus)). In one or more embodiments of the present disclosure, the vectors are pUC57 vectors and/or retroviral vectors MP71.

The microorganisms mentioned herein may comprise bacteria, yeasts, algae, or fungi. Among them, bacteria may come from genera such as *Escherichia sp., Erwinia sp., Agrobacterium sp., Flavobacterium sp., Alcaligenes sp., Pseudomonas sp., and Bacillus sp.,* but are not limited to these. Yeasts may come from genera such as *Saccharomyces sp., Pichia sp., Yarrowia sp., Candida sp., Schizosaccharomyces sp., Hansenula sp.,* and *Kluyveromyces sp.,* but are not limited to these. Algae may come from genera such as *Fucus sp., Achnanthes sp., Amphiprora sp., Amphora sp., Ankistrodesmus sp., Asteromonas sp.,* and *Boekelovia sp.,* but are not limited to these. Fungi may come from genera such as *Fusarium sp., Rhizoctonia sp., Verticillium sp., Penicillium sp., Aspergillus sp.,* and *Cephalosporium sp.,* but are not limited to these. In one or more embodiments of the present disclosure, the microorganism is *Escherichia coli* DH5α.

The recombinant vectors mentioned herein refer to recombinant vector DNA molecules constructed by connecting exogenous target genes with vectors in vitro. They can be constructed in any suitable manner, as long as the constructed recombinant vectors can carry the exogenous target genes into recipient cells and provide them with the ability to replicate, integrate, amplify, and/or express within the recipient cells. In one or more embodiments of the present disclosure, the recombinant vector is the recombinant retroviral vector MP71-CLL1-VHH-1 CAR.

The recombinant retroviral vector MP71-CLL1-VHH-1 CAR is obtained by replacing the fragment (small fragment) between the *Not*I and *EcoR*I recognition sites of the retroviral vector MP71 with a DNA fragment whose nucleotide sequence is SEQ ID No.2 in the sequence listing, while keeping the other nucleotide sequences of the retroviral vector MP71 unchanged, resulting in a recombinant expression vector.

The recombinant microorganisms mentioned herein refer to microorganisms whose genes have been manipulated and modified to result in changes in function, such as recombinant microorganisms obtained by introducing exogenous target genes or recombinant vectors into target microorganisms, or recombinant microorganisms obtained by directly editing the endogenous genes of target microorganisms. In one or more embodiments of the present disclosure, the recombinant microorganism is a recombinant strain obtained by introducing the recombinant retroviral vector MP71-CLL1-VHH-1 CAR into *Escherichia coli* DH5α, which contains the DNA molecule shown in SEQ ID No.2.

The CLL1-CAR-T cells mentioned herein are recombinant cells obtained by stably expressing the constructed chimeric antigen receptor (CAR) gene in T cells through genetic engineering and in vitro recombination methods. Furthermore, the CLL1-CAR-T cells mentioned herein may be CLL1-VHH-1 CAR-T cells obtained by stably expressing the CLL1-VHH-1 CAR gene (SEQ ID No.2) in T cells.

Specifically, the CLL1-VHH-1 CAR-T cells can be recombinant cells obtained by packaging the recombinant retroviral vector MP71-CLL1-VHH-1 CAR with packaging cells to generate recombinant retroviruses and then introducing these recombinant retroviruses into T cells. The packaging cells can be Phoenix Ecotropic (ECO) cells and PG13 cells.

The CLL1-VHH-1 CAR-T cells contain and/or express the CLL1-VHH-1 CAR gene (SEQ ID No.2).

The CLL1-VHH-1 CAR-T cells described herein possess at least one of the following characteristics:

F1) secretion of the specific effector molecule IFN-γ;
F2) specific killing of CLL1$^+$ tumor cells; and
F3) inhibition of the proliferation of CLL1$^+$ tumor cells.

This disclosure also provides the application of the chimeric antigen receptor CLL1-VHH-1 CAR in the preparation of the aforementioned CLL1-VHH-1 CAR-T cells.

This disclosure also provides a pharmaceutical composition, which may contain any of the CLL1-CAR-T cells described herein.

The pharmaceutical composition can be used for the prevention or treatment of diseases related to the CLL1 target.

Furthermore, the pharmaceutical composition may also comprise one or more pharmaceutically acceptable carriers.

Furthermore, the pharmaceutically acceptable carriers, selected from excipients, stabilizers, suspending agents, diluents, etc., are well-known to those skilled in the art.

This disclosure also provides any of the following applications of any of the CLL1-CAR-T cells, the chimeric antigen receptor, the biological material, or the pharmaceutical composition described herein:

D1) application in the preparation of drugs for the prevention or treatment of tumors, or application in the prevention or treatment of tumors;
D2) application in the preparation of drugs for the prevention or treatment of diseases related to the CLL1 target, or application in the prevention or treatment of diseases related to the CLL1 target.

In the aforementioned applications, the diseases related to the CLL1 target can be CLL1-positive cancers (i.e., cancers expressing CLL1).

Furthermore, the CLL1-positive cancers can be Acute Myeloid Leukemia (AML), Myelodysplastic Syndromes (MDS), or Chronic Myeloid Leukemia (CML, also known as Chronic Granulocytic Leukemia).

This disclosure also provides a method for preparing any of the CLL1-CAR-T cells described herein, which may comprise introducing any of the nucleic acid molecules described herein into T cells for stable expression to obtain the CLL1-CAR-T cells.

Furthermore, the method may comprise the following steps:

(1) constructing the CLL1-VHH-1 CAR gene (SEQ ID No.2) between the *Not*I and *EcoR*I recognition sites of the retroviral vector MP71 to obtain the recombinant retroviral vector MP71-CLL1-VHH-1 CAR;
(2) introducing the recombinant retroviral vector MP71-CLL1-VHH-1 CAR into packaging cells for packaging to obtain recombinant retroviruses; and
(3) infecting human T cells with the recombinant retroviruses to obtain recombinant cells, namely CLL1-VHH-1 CAR-T cells.

Furthermore, the packaging cells can be Phoenix Ecotropic (ECO) cells and PG13 cells.

This disclosure also provides a method for the prevention or treatment of diseases related to the CLL1 target, which may comprise administering any of the CLL1-CAR-T cell or the pharmaceutical composition described herein to a subject suffering from diseases related to the CLL1 target.

Furthermore, the diseases related to the CLL1 target can be CLL1-positive cancers.

Furthermore, the CLL1-positive cancers can be Acute Myeloid Leukemia, Myelodysplastic Syndromes, or Chronic Myeloid Leukemia.

CLL1, described herein as C-type lectin-like molecule 1, is a type II transmembrane glycoprotein that plays an important role in immune regulation as an inhibitory receptor. CLL1 is present in myeloid cells of peripheral blood and bone marrow, as well as in most Acute Myeloid Leukemia (AML) cells. It is also expressed on the $CD34^+CD38^-$ stem cells of most AML cases, while not expressed on the $CD34^+CD38^-$ stem cells of normal individuals. Due to its unique expression pattern, CLL1 has become a potential target for the treatment and diagnosis of AML.

This disclosure first designed a chimeric antigen receptor (SEQ ID No.1) for constructing CLL1-CAR-T cells, with its structure shown in FIG. 1. Furthermore, CAR-T cells (CLL1-VHH-1 CAR-T cells) targeting CLL1 with a novel structure are prepared. Experimental results show that the CLL1-VHH-1 CAR-T cells of this disclosure can effectively secrete the T-cell-specific effector molecule IFN-$\gamma$, specifically kill $CLL1^+$ target cells, and exhibit good in vivo tumor-killing activity. They can not only significantly inhibit the proliferation of tumor cells in mice but also significantly prolong the survival time of mice. On days D28 and D35 after injection into mice, the tumor tissues in the mice almost completely disappeared. The CLL1-VHH-1 CAR-T cells of this disclosure have good anti-tumor ability and can be used for the immunotherapy of diseases related to the CLL1 target (such as Acute Myeloid Leukemia, Myelodysplastic Syndromes, or Chronic Myeloid Leukemia), with broad clinical application prospects.

## BRIEF DESCRIPTION OF THE DRAWINGS

FIG. 1 is a schematic diagram of the molecular structure of CLL1-VHH-1 CAR.
FIG. 2 shows the detection of CAR expression in CLL1-VHH-1 CAR-T cells.
FIG. 3 depicts the cellular functional assay for IFN-$\gamma$ secretion by CLL1-VHH-1 CAR-T cells.
FIG. 4 illustrates the cytotoxicity assay of CLL1-VHH-1 CAR-T cells.
FIG. 5 presents the in vivo tumor-killing activity assay of CLL1-VHH-1 CAR-T cells.

## DETAILED DESCRIPTION

The present disclosure will be further described in detail below in conjunction with specific embodiments. The examples provided are solely for the purpose of illustrating the disclosure and are not intended to limit its scope. The examples presented here can serve as a guide for further improvements by those of ordinary skill in the art and do not, in any way, constitute a restriction on the disclosure.

Unless otherwise specified, the experimental methods in the following examples are conventional methods, carried out in accordance with the techniques or conditions described in the literature within the field or as per the product instructions. The materials, reagents, etc., used in the following examples can be obtained commercially unless otherwise specified.

The retroviral vector MP71 used in the following examples is documented in the following literature: Engels B, Cam H, et al. Retroviral Vectors for High-Level Transgene Expression in T Lymphocytes [J]. Human Gene Therapy, 2003, 14(12):1155-1168. The public can obtain this biological material from the applicant. This biological material is intended solely for the purpose of repeating the experiments of the present disclosure and should not be used for any other purposes.

The human peripheral blood mononuclear cells (Peripheral blood mononuclear cell, PBMC) used in the following examples are derived from the venous blood of healthy volunteers.

The U937 cells (CLL1-positive cells) used in the following examples are acute myeloid leukemia cells, a product of Beijing Bio-Biobw Biotech Co., Ltd., with the product number bio-73180.

## Example 1: Design of the Chimeric Antigen Receptor (CAR) for Constructing CLLI-CAR-T Cells

In this example, the chimeric antigen receptor (CAR) designed for constructing CLL1-CAR-T cells is named CLL1-VHH-1 CAR. From the N-terminus to the C-terminus, it sequentially consists of a signal peptide (Signal), a single-domain antibody (CLL1-VHH-1), a CD8a hinge region (Hinge), a CD8a transmembrane region, a co-stimulatory domain 4-1BB, and an activation domain CD3ζ (as shown in FIG. 1). Among these components, the single-domain antibody CLL1-VHH-1 is a single-domain antibody that specifically binds to the CLL1 protein. It serves as the extracellular antigen-binding region of the CAR, responsible for recognizing and binding to the tumor surface antigen CLL1. The N-terminus of the antigen-binding region is equipped with a signal peptide (Signal) that guides the peptide chain of the antigen-binding region to be transferred outside the cell, thereby enabling it to recognize the tumor cell surface antigen CLL1. The CD8a hinge region (Hinge) is a peptide chain of the CAR located extracellularly, connecting the extracellular antigen-binding region and the CD8a transmembrane region. The CD8a transmembrane region is used to anchor the single-domain antibody CLL1-VHH-1 onto the cell membrane. The intracellular signaling region comprises the co-stimulatory domain 4-1BB and the activation domain CD3ζ, which work together to stimulate T cells, activate intracellular signals, enable T cells to continuously proliferate and release cytokines, enhance the anti-tumor ability of T cells, and simultaneously exert T cell signal transduction functions to promote a more robust and sustained T cell response.

The designed amino acid sequence of CLL1-VHH-1 CAR is set forth in SEQ ID No.1 (378 aa). The amino acid sequence of the signal peptide (Signal) is located at positions 1-21 of SEQ ID No.1. The amino acid sequence of the single-domain antibody CLL1-VHH-1 is located at positions 22-150 of SEQ ID No.1. The amino acid sequence of the CD8a hinge region (Hinge) is located at positions 151-195 of SEQ ID No.1. The amino acid sequence of the CD8a transmembrane region is located at positions 196-219 of SEQ ID No.1. The amino acid sequence of the co-stimulatory domain 4-1BB is located at positions 220-266 of SEQ ID No.1. The amino acid sequence of the activation domain CD3ζ is located at positions 267-378 of SEQ ID No.1.

The coding gene for CLL1-VHH-1 CAR is the CLL1-VHH-1 CAR gene, and its nucleotide sequence is set forth in SEQ ID No.2 (1134 bp). The nucleotide sequence of the signal peptide (Signal) is located at positions 1-63 of SEQ ID No.2. The nucleotide sequence of the single-domain antibody CLL1-VHH-1 is located at positions 64-450 of SEQ ID No.2. The nucleotide sequence of the CD8a hinge region (Hinge) is located at positions 451-585 of SEQ ID No.2. The nucleotide sequence of the CD8a transmembrane region is located at positions 586-657 of SEQ ID No.2. The nucleotide sequence of the co-stimulatory domain 4-1BB is located at positions 658-798 of SEQ ID No.2. The nucleotide sequence of the activation domain CD3ζ is located at positions 799-1134 of SEQ ID No.2.

**Example 2: Preparation of CLL1-VHH-1 CAR-T Cells**

The CLL1-CAR-T cells constructed using the CLL1-VHH-1 CAR designed in Example 1 are named CLL1-VHH-1 CAR-T cells. Specifically, the CLL1-VHH-1 CAR gene (SEQ ID No.2) from Example 1 is stably expressed in T cells to obtain T cells expressing the CLL1-VHH-1 CAR gene, which are the CLL1-VHH-1 CAR-T cells. The steps are shown as follows.

2-1: Construction of the Recombinant Retroviral Vector

(1) The recombinant vector pUC57-CLL1-VHH-1 CAR is digested with *Not*I (NEB) and *EcoR*I (NEB) double enzymes, followed by gel extraction to recover the target gene fragment. The recombinant vector pUC57-CLL1-VHH-1 CAR is a recombinant vector obtained by cloning the CLL1-VHH-1 CAR gene (SEQ ID No.2) into the pUC57 vector, synthesized and provided by Tsingke Biotech Co., Ltd.

(2) The retroviral vector MP71 is digested with *Not*I and *EcoR*I double enzymes, followed by gel extraction to recover the large vector fragment.

(3) The above target gene fragment and the large vector fragment is ligated using T4 ligase (NEB) to obtain the recombinant retroviral vector MP71-CLL1-VHH-1 CAR carrying the CLL1-VHH-1 CAR gene.

(4) The recombinant retroviral vector MP71-CLL1-VHH-1 CAR is transformed into competent *Escherichia coli* DH5α, extract and purify the plasmid using the Qiagen plasmid purification kit to obtain the MP71-CLL1-VHH-1 CAR plasmid.

The recombinant retroviral vector MP71-CLL1-VHH-1 CAR (i.e., the MP71-CLL1-VHH-1 CAR plasmid) is a recombinant expression vector obtained by replacing the fragment (small fragment) between the *Not*I and *EcoR*I recognition sites of the retroviral vector MP71 with a DNA fragment having the nucleotide sequence of SEQ ID No.2 in the sequence listing, while keeping the other nucleotide sequences of the retroviral vector MP71 unchanged.

2-2: Retroviral Packaging

The MP71-CLL1-VHH-1 CAR plasmid prepared in step 2-1 is introduced into packaging cells for packaging to complete virus assembly and obtain retroviruses. The specific steps for virus packaging are shown as follows.

a) Day 1: Phoenix Ecotropic (ECO) cells should be less than 20 passages and not overly confluent. The cells are placed at a density of $0.6 \times 10^6$/ml, 10 mL of DMEM medium is added to a 10 cm dish, then the cells are thoroughly mixed, and cultured at 37°C overnight.

b) Day 2: When the ECO cell confluence reaches approximately 90%, transfection (usually around 14-18 hours after plating) is performed. 12.5 $\mu$g of plasmid, 250 $\mu$L of 1.25 M $CaCl_2$, 1 mL of $H_2O$ are prepared to achieve a total volume of 1.25 mL. An equal volume of 2 × HBS is added to another tube, vortexed and mixed for 20 seconds while the plasmid complex is being added. The mixture is gently added along the edge into the ECO dish, cultured at 37°C for 4 hours, after which the medium is removed, the dish is washed once with PBS, and fresh pre-warmed medium is added.

c) Day 4: the supernatant 48 hours is collected after transfection, it is filtered with a 0.45 $\mu$m filter to obtain the retroviral solution, aliquoted and stored at -80°C.

d) 1.2 mL of a 15 $\mu$g/mL RetroNectin coating solution is added to each well of an NTC 6-well plate and incubated at 4°C overnight.

e) The blocking solution is carefully aspirated, washed with 2 mL/well of PBS, 5 mL of the above virus solution to each well is added, centrifuged at 32°C and 2000 × g for 2 hours, and the unbound virus supernatant is aspirated.

f) Logarithmic-phase PG13 cells is taken, rinsed once with 10 mL of PBS, 1 mL of 0.25% recombinant trypsin is added, and it is let stand at room temperature for 2-3 minutes.

g) 5 mL of complete medium containing 10% FBS is added to terminate digestion, centrifuged at 1500 rpm for 5 minutes.

h) The supernatant is aspirated, the cell density to $0.5 \times 10^5$ cells/mL is adjusted with complete medium, 3 mL/well is added to the above virus-coated NTC 6-well plate, so that the final cell number is $1.5 \times 10^5$ cells/well.

i) It is centrifuge at 1000 rpm for 1 minute, cultured at 37°C and 5% $CO_2$ for 48 hours.

j) After 1-2 passages, it is transferred to a T175 culture flask and cultured with DEME medium containing 12% FBS for 2 days.

k) it is replaced with new DEME medium containing 12% FBS, it is continued to culture for 48 hours, the supernatant is collected, it is filtered with a 0.45 $\mu$m filter to obtain the retroviral solution, aliquoted and stored at -80°C.

2-3: Retroviral Infection of Human T Cells

a) Frozen healthy human peripheral blood PBMCs are thawed, the cell density is adjusted to $1 \times 10^6$-$2 \times 10^6$ cells/mL with RPMI-1640 medium containing 10% fetal bovine serum (FBS).

b) PBMCs are collected using Ficoll separation solution (Tianjin Haoyang), CD3$^+$ T cells is isolated by magnetic bead method, and T cells is activated by adding clinical-grade Dynabeads® Human T Expander CD3/CD28 magnetic beads (Invitrogen) at a ratio of magnetic beads: CD3$^+$ cells = 3:1.

c) On the second day after T cell activation, non-tissue-treated culture plates are coated with RetroNectin (TAKARA) diluted to a final concentration of 15 $\mu$g/mL in PBS, with 1.2 mL per well of a 6-well plate. It is kept in the dark and incubated at 4°C overnight for later use.

d) On the second day after T cell activation culture, the coated 6-well plate is taken out, the coating solution is aspirated, and the plate is washed once with PBS.

e) The retroviral solution prepared in step 2-2 is added to the wells, with 5-6 mL per well, is centrifuged at 32°C and 2000 × g for 2 hours. 3 mL of fresh complete medium containing 500 U/mL hIL-2 (human interleukin-2) is added to each well and continue to culture for 1 day.

f) After cell infection, the cell density is observed daily, and T cell culture medium containing 100 U/mL IL-2 is added in a timely manner to maintain the T cell density at around $5 \times 10^5$/mL for cell expansion.

g) Thus, CAR-T cells infected with the retrovirus prepared in step 2-2, namely T cells expressing the CLL1-VHH-1 CAR gene with the nucleotide sequence of SEQ ID No.2, are obtained and named CLL1-VHH-1 CAR-T cells.

The constructed CLL1-VHH-1 CAR-T cells and CTR T cells (i.e., T cells without viral transfection, used as a control) are cultured at 37°C in RPMI-1640 medium containing 10% fetal bovine serum (FBS), and this day is recorded as D0. The cells are cultured until D10 for various functional tests.

**Example 3: Detection of CAR Expression in CLL1-VHH-1 CAR-T Cells**

The constructed CLL1-VHH-1 CAR-T cells express the chimeric antigen receptor CLL1-VHH-1 CAR with the amino acid sequence of SEQ ID No.1. The steps for detecting CAR expression are shown as follows:

1.After centrifuging the cells (1500 rpm × 5 min), the supernatant is discarded. 200 $\mu$L of FACS buffer (1 × PBS

containing 0.1% NaN$_3$ and 2% FBS) is added to each well of a 96-well round-bottom plate to resuspend the cells, followed by centrifugation at 1500 rpm for 5 min.

2.60 μL of the prepared fluorescently labeled anti-human CD4-APC Cy7/CLL1-FITC is added to each well, mixed well, and incubated at 4°C for 30 min.

3.200 μL of FACS buffer is added to each well, followed by centrifugation at 1500 rpm for 5 min.

4.The supernatant is discarded, the cells are resuspended in 400 μL of FACS buffer, and they are transferred to flow cytometry reading tubes. The cells are read using a flow cytometer (BD Canto-II) and the percentage of CLL1 antibody in the CLL1-VHH-1 CAR-T cells is analyzed.

The detection results are shown in FIG. 2. The CLL1-VHH-1 CAR-T cells can effectively express the CLL1 antibody (i.e., the single-domain antibody CLL1-VHH-1), indicating that the expression of CAR molecules in the CLL1-VHH-1 CAR-T cells meets the expected design.

**Example 4: Functional Detection of the Secretion of the Specific Effector Molecule IFN-γ by CLL1-VHH-1 CAR-T Cells**

1.The cell density of the test cells, CLL1-VHH-1 CAR-T cells and CTR T cells (i.e., non-virus-transfected T cells used as a control), is adjusted to $2 \times 10^6$ cells/mL. 100 μL is added to a 96-well U-bottom plate. U937 cells at a ratio of test cells is added to target cells of 1:1. Brefeldin A (MedChemExpress, HY-16592) is added to each well at a final concentration of 5 μg/mL, and incubated in a 37°C incubator for 6 hours.

CTR + U937: 1 mL of a total of $1 \times 10^6$ non-virus-transfected T cells (CTR T cells) and 1 mL of a total of $1 \times 10^6$ U937 cells are added to each well.
CLL1-VHH-1 CAR-T + U937: 1 mL of a total of $1 \times 10^6$ CLL1-VHH-1 CAR-T cells and 1 mL of a total of $1 \times 10^6$ U937 cells are added to each well.
2. After incubation, perform flow cytometry staining. The operational steps are shown as follows:

(1) After centrifuging the cells (1500 rpm × 5 min), the supernatant is discarded. 200 μL of FACS buffer (1 × PBS containing 0.1% NaN$_3$ and 2% FBS) is added to each well to resuspend the cells, followed by centrifugation at 1500 rpm for 5 min. This step is repeated twice.
(2) 60 μL of the prepared fluorescently labeled CLL1(rp)-PE is added to each well, mixed well, and incubated in the dark at room temperature for 10 min.
(3) 200 μL of FACS buffer is added to each well, centrifuged at 1500 rpm for 5 min, and then the supernatant is discarded.
(4) 150 μL of Cytofix/Cytoperm (BD, catalog number 55472) is added to each well, mixed well, and incubated in the dark at room temperature for 15 min.
(5) After centrifuging at 1500 rpm for 5 min and discarding the supernatant, 200 μL of Perm/Wash buffer (BD, catalog number 554723) is added to each well, mixed well, and centrifuged at 1500 rpm for 5 min. It is washed by centrifugation twice.
(6) 20 μL of diluted APC-labeled anti-human IFN-γ (Biolegend, catalog number 506510) is added to each well, mixed well, and incubated in the dark at room temperature for 20 min.
(7) 200 μL of Perm buffer is added to each well and centrifuged at 1500 rpm for 5 min. After discarding the supernatant, the cells are resuspend in 400 μL of FACS buffer and transferred to flow cytometry reading tubes. The cells are analyzed using a flow cytometer (BD Canto-II) to determine the percentage of the functional effector molecule IFN-γ in CLL1-VHH-1 CAR-T cells and control cells (CTR T cells).

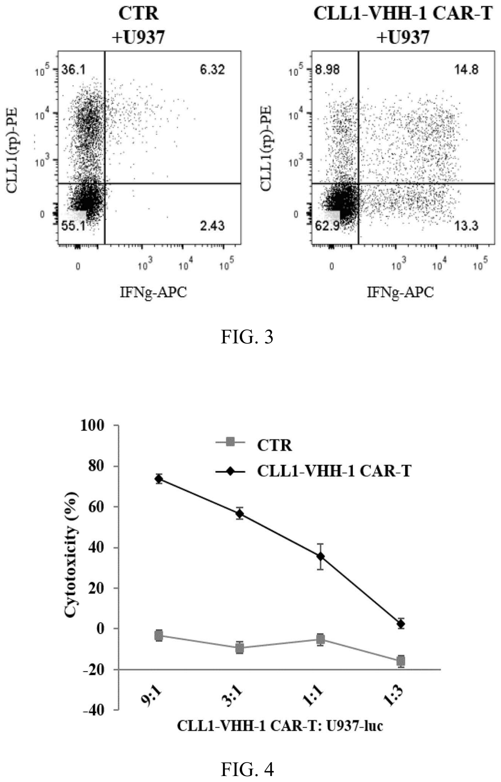

The detection results, as shown in FIG. 3, indicate that after co-culturing with U937 target cells, CLL1-VHH-1 CAR-T cells can effectively secrete the T-cell-specific effector molecule IFN-γ.

**Example 5: Detection of the Cytotoxic Effect of CLL1-VHH-1 CAR-T Cells**

1.Cell density of the effector cells, CLL1-VHH-1 CAR-T cells and CTR T cells (i.e., non-virus-transfected T cells used as a control), are adjusted to $2 \times 10^5$ cells/mL. 100 μL is added to a 96-well U-bottom plate. D-firefly luciferin sodium salt (Yeasen Biotechnology, catalog number 40901ES08, stored at a concentration of 100 mg/mL) is added at a final concentration of 100 μg/mL. It is repeated for three wells.

2.Target cells U937-luc are added at different effector-to-target ratios (9:1, 3:1, 1:1, 1:3), with the target cell density being $2 \times 10^4$ cells under the 1:1 condition. It is incubated at 37°C for 16 h.

3.The fluorescence values is measured using a TECAN spark microplate reader. The specific cytotoxic activity (Cytotoxicity) of CLL1-VHH-1 CAR-T cells is calculated by taking the average of the three replicate wells:

$$\text{Specific lysis \%} = 100 - 100 \times (E_{exp} - E_{min}) / (T_{max} - T_{min})$$

$E_{exp}$: RLU (Relative Light Unit) value when effector cells and target cells are co-cultured.

$E_{min}$: RLU value of spontaneous death of effector cells in the absence of target cells.

$T_{max}$: RLU value of spontaneous death of target cells in the absence of effector cells.

$T_{min}$: RLU value under conditions of maximum killing rate.

The detection results, as shown in FIG. 4, indicate that CLL1-VHH-1 CAR-T cells can effectively and specifically kill CLL1+ target cells (U937 cells), while the control CTR T cells have no cytotoxic effect on CLL1+ target cells.

**Example 6: Detection of in Vivo Tumor-Killing Activity of CLL1-VHH-1 CAR-T Cells**

1.Eight female NSG mice (Shanghai Model Organisms Center, Inc., catalog number NM-NSG-001, aged 8 - 10 weeks) are each intravenously inoculated with $2.0 \times 10^6$ U937-Luc-GFP cells via the tail vein.

2.Six days after inoculation, the mice are randomly divided into two groups. Each mouse received an intravenous injection of $4.0 \times 10^6$ CLL1-VHH-1 CAR-T or CTR T (i.e., non-virus-transfected T cells used as a control) cells via the tail vein, and this day is designated as Day 0 (D0).

3.Tumor luminescence signals are captured using a Photo Acquisition small animal imager on Days 7 (D7), 14 (D14), 21 (D21), 28 (D28), and 35 (D35).

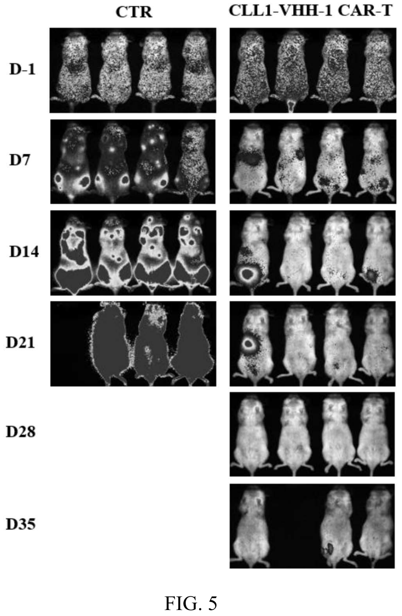

The detection results, as shown in Table 1 and FIG. 5, indicate that compared to the CTR control group, CLL1-VHH-1 CAR-T cells not only significantly inhibited the proliferation of tumor cells in mice but also significantly prolonged the survival time of the mice. By D28 and D35, the tumor tissues in the mice have almost completely disappeared.

Table 1: Bioluminescence Intensity (P/S) of Tumor Cells in Tumor-Bearing Mice

| category | serial number | D-1 | D7 | D14 | D21 | D28 | D35 |
|---|---|---|---|---|---|---|---|
| CTR | 1 | 3.57E+06 | 7.29E+07 | 2.41E+09 | N/A | N/A | N/A |
| | 2 | 2.42E+06 | 4.77E+07 | 1.01E+09 | 6.39E+10 | N/A | N/A |
| | 3 | 2.87E+06 | 4.86E+07 | 2.41E+09 | 8.71E+09 | N/A | N/A |
| | 4 | 3.16E+06 | 1.04E+07 | 1.21E+09 | 3.42E+10 | N/A | N/A |
| CLL1-VHH-1 CAR-T | 1 | 1.70E+07 | 2.03E+06 | 4.71E+07 | 3.95E+07 | 7.17E+05 | 1.94E+06 |
| | 2 | 2.79E+07 | 6.97E+05 | 2.13E+06 | 2.37E+06 | 5.54E+05 | N/A |
| | 3 | 1.79E+07 | 2.11E+06 | 2.46E+06 | 2.67E+06 | 2.50E+06 | 3.13E+07 |
| | 4 | 1.50E+07 | 3.37E+06 | 3.28E+06 | 2.19E+06 | 4.14E+05 | 1.22E+06 |

The present disclosure has been described in detail above. For those skilled in the art, the disclosure can be implemented within a broad range under equivalent parameters, concentrations, and conditions without deviating from its purpose and scope, and without the need for unnecessary experimentation. Although specific embodiments of the disclosure have been provided, it should be understood that further modifications to the disclosure are possible. In summary, in accordance with the principles of the present disclosure, this application is intended to encompass any alterations, uses, or improvements to the disclosure, including changes made using conventional techniques known in the art that fall outside the scope of what has been disclosed in this application. The application of certain essential features may be carried out within the scope of the accompanying claims below.

Industrial Applicability

The CAR-T cells targeting CLL1 with a novel structure (CLL1-VHH-1 CAR-T cells) designed in the present disclosure are capable of effectively secreting the T-cell-specific effector molecule IFN-$\gamma$ and specifically killing CLL1$^+$ target cells with high efficiency. These cells exhibit excellent in vivo tumor-killing activity, not only significantly inhibiting the proliferation of tumor cells in mice but also markedly prolonging the survival time of mice. The CLL1-VHH-1 CAR-T cells of the present disclosure demonstrate strong antitumor capabilities and can be utilized for the immunotherapy of diseases associated with the CLL1 target (such as acute myeloid leukemia, myelodysplastic syndromes, or chronic myeloid leukemia), offering broad prospects for clinical application.

**Claims**

1.  A CLL1-CAR-T cell, comprising a chimeric antigen receptor, wherein the chimeric antigen receptor comprises a single-domain antibody, a hinge region, a transmembrane region, and an intracellular signaling region, and an amino acid sequence of the single-domain antibody corresponds to positions 22-150 of SEQ ID No.1.

2.  The CLL1-CAR-T cell according to claim 1, wherein the intracellular signaling region comprises a co-stimulatory domain 4-1BB and an activation domain CD3$\zeta$, an amino acid sequence of the 4-1BB corresponds to positions 220-266 of SEQ ID No.1, and an amino acid sequence of the CD3$\zeta$ corresponds to positions 267-378 of SEQ ID No.1.

3.  The CLL1-CAR-T cell according to claim 1 or 2, wherein the hinge region is a CD8a hinge region with an amino acid sequence corresponds to positions 151-195 of SEQ ID No.1, and the transmembrane region is a CD8a transmembrane region with an amino acid sequence corresponds to positions 196-219 of SEQ ID No.1.

4.  The CLL1-CAR-T cell according to any one of claims 1-3, wherein the chimeric antigen receptor comprises any one of the following:

    A1) a protein with an amino acid sequence set forth in SEQ ID No.1 or positions 22-378 of SEQ ID No.1;
    A2) a protein obtained by substitution and/or deletion and/or addition of amino acid residues in the amino acid sequence set forth in SEQ ID No.1 or positions 22-378 of SEQ ID No.1, which has more than 80% identity with the protein in A1) and the same function;
    A3) a fusion protein obtained by connecting a tag or signal peptide to the N-terminus and/or C-terminus of A1 or A2) with the same function as A1).

5.  A chimeric antigen receptor according to any one of claims 1-4.

6.  A biological material, wherein the biological material is any one of the following:

    B1) a nucleic acid molecule encoding the chimeric antigen receptor according to claim 5;
    B2) an expression cassette comprising the nucleic acid molecule in B1);
    B3) a recombinant vector comprising the nucleic acid molecule in B1) or a recombinant vector comprising the expression cassette in B2);
    B4) a recombinant microorganism comprising the nucleic acid molecule in B1), or a recombinant microorganism comprising the expression cassette in B2), or a recombinant microorganism comprising the recombinant vector in B3).

7.  The biological material according to claim 6, wherein the nucleic acid molecule in B1) is any one of the following:

    C1) a DNA molecule with a nucleotide sequence or coding sequence set forth in positions 64-1134 of SEQ ID No.2;

C2) a DNA molecule with more than 75% identity with the nucleotide sequence defined in C1) and the same function;

C3) a DNA molecule with a nucleotide sequence or coding sequence set forth in SEQ ID No.2;

C4) a DNA molecule with more than 75% identity with the nucleotide sequence in C3) and the same function.

8. A pharmaceutical composition, wherein the pharmaceutical composition comprises the CLL1-CAR-T cell according to any one of claims 1-4.

9. Any one of the following applications of the CLL1-CAR-T cell according to any one of claims 1-4, the chimeric antigen receptor according to claim 5, the biological material according to claim 6 or 7, or the pharmaceutical composition according to claim 8:

D1) application in preparing a drug for preventing or treating tumors, or application in preventing or treating tumors;

D2) application in preparing a drug for preventing or treating diseases related to the CLL1 target, or application in preventing or treating diseases related to the CLL1 target.

10. The application according to claim 9, wherein the diseases related to the CLL1 target are CLL1-positive cancers.

11. The application according to claim 10, wherein the CLL1-positive cancers are acute myeloid leukemia, myelodysplastic syndrome, or chronic myeloid leukemia.

12. A method for preparing the CLL1-CAR-T cell according to any one of claims 1-4, wherein the method comprises transferring the nucleic acid molecule in claim 6 or 7 into T cells for stable expression to obtain the CLL1-CAR-T cell.

13. A method for preventing or treating diseases related to the CLL1 target, wherein the method comprises administering the CLL1-CAR-T cell according to any one of claims 1-4 or the pharmaceutical composition according to claim 8 to a subject suffering from diseases related to the CLL1 target.

14. The method according to claim 13, wherein the diseases related to the CLL1 target are CLL1-positive cancers.

15. The method according to claim 14, wherein the CLL1-positive cancers are acute myeloid leukemia, myelodysplastic syndrome, or chronic myeloid leukemia.

FIG. 1

FIG. 2

FIG. 3

FIG. 4

FIG. 5

## INTERNATIONAL SEARCH REPORT

| | International application No. |
|---|---|
| | **PCT/CN2024/070562** |

**A. CLASSIFICATION OF SUBJECT MATTER**

C12N 5/10(2006.01)i; C07K 19/00(2006.01)i; C12N 15/62(2006.01)i; A61K 39/00(2006.01)i; A61P 35/02(2006.01)i

According to International Patent Classification (IPC) or to both national classification and IPC

**B. FIELDS SEARCHED**

Minimum documentation searched (classification system followed by classification symbols)

IPC: C12N,C07K,A61K, A61P

Documentation searched other than minimum documentation to the extent that such documents are included in the fields searched

Electronic data base consulted during the international search (name of data base and, where practicable, search terms used)

CNABS, WPABSC, CNTXT, ENTXTC, DWPI, WPABS, VEN, ENTXT, CJFD, CNKI, 万方, WANFANG, PubMed, Genbank, 中国专利生物序列检索系统, China Patent Biological Sequence Search System, REGISTRY: C型凝集素样分子1, C型凝集素结构域家族12成员A, 嵌合抗原受体, 急性髓细胞/髓性/髓系/骨髓性白血病, 骨髓增生异常综合征, 慢性髓细胞/髓系/粒细胞白血病, 单域抗体, 纳米抗体, 重链抗体, CLL1-CAR-T, CLL1-VHH-1, Chimeric Antigen Receptor, CAR, CAR-T, C-type lectin like molecule, CLL1, CLL-1, C-type lectin domain family 12 member A, CLEC12A, myeloid inhibitory C-type lectin-like receptor, MICL, dendritic cell-associated lectin 2, DCAL2, DCAL-2, killer cell lectin like receptor-1, KLRL1, CD371, Acute Myeloid Leukemia, AML, Myelodysplastic Syndrome, MDS, Chronic Myeloid Leukemia, CML, sdAb, single domain antibody, nanobody, heavy chain antibody, hcAb, SEQ ID NO.1的序列检索, search for SEQ ID NO. 1

**C. DOCUMENTS CONSIDERED TO BE RELEVANT**

| Category* | Citation of document, with indication, where appropriate, of the relevant passages | Relevant to claim No. |
|---|---|---|
| PX | CN 116515765 A (CARBIOGENE THERAPEUTICS CO., LTD.) 01 August 2023 (2023-08-01)<br>see claims 1-10, and description, paragraphs [0053]-[0066] | 1-15 |
| A | CN 115772222 A (CARBIOGENE THERAPEUTICS CO., LTD.) 10 March 2023 (2023-03-10)<br>see abstract, and description, paragraph [0006] | 1-15 |
| A | CN 112789293 A (NANJING LEGEND BIOTECH CO., LTD.) 11 May 2021 (2021-05-11)<br>see abstract, and description, paragraphs [0016] and [0032] | 1-15 |
| A | US 2022354890 A1 (LI, Guangchao) 10 November 2022 (2022-11-10)<br>see claims 1-15 | 1-15 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **30 March 2024** | **10 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/070562** |

### C.    DOCUMENTS CONSIDERED TO BE RELEVANT

| Category* | Citation of document, with indication, where appropriate, of the relevant passages | Relevant to claim No. |
|---|---|---|
| A | 吴卓琳 等 (WU, Zhuolin et al.). "CAR-T治疗急性髓系白血病的研究进展 (Research Progress of CAR-T in the Treatment of Acute Myeloid Leukemia)" *临床内科杂志 (Journal of Clinical Internal Medicine)*. Vol. 39, No. (9), 15 September 2022 (2022-09-15), pp. 594-597 <br>    see page 595, left-hand column, paragraph 2 | 1-15 |
| A | WANG, Jinghua et al. "CAR-T Cells Targeting CLL-1 as an Approach to Treat Acute Myeloid Leukemia" <br>*Journal of Hematology & Oncology*, Vol. vol. 11, 10 January 2018 (2018-01-10), Article number 7, pages 1-13 <br>    see abstract | 1-15 |

Form PCT/ISA/210 (second sheet) (July 2022)

**INTERNATIONAL SEARCH REPORT**

| International application No. |
| --- |
| **PCT/CN2024/070562** |

---

**Box No. I      Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

   ☐  accompanied by a statement to the effect that the sequence listing does not go beyond the disclosure in the international application as filed.

2. ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/070562** |

| **Box No. II** | **Observations where certain claims were found unsearchable (Continuation of item 2 of first sheet)** |
| --- | --- |

This international search report has not been established in respect of certain claims under Article 17(2)(a) for the following reasons:

1. ☑ Claims Nos.: **13-15**
   because they relate to subject matter not required to be searched by this Authority, namely:

   Claims 13-15 relate to a method for treating a human or animal body by therapy, which falls within subject matter for which no international search is required as defined in PCT Rule 39.1(iv). However, the search for claims 13-15 has been carried out on the basis of the modified subject matter that is reasonably expected, that is, the use of the CLL1-CAR-T cell of any one of claims 1-4 or the pharmaceutical composition of claim 8 in the preparation of a drug for preventing or treating CLL1 target-related diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/070562** |

| Patent document cited in search report | | | Publication date (day/month/year) | Patent family member(s) | | | Publication date (day/month/year) |
|---|---|---|---|---|---|---|---|
| CN | 116515765 | A | 01 August 2023 | None | | | |
| CN | 115772222 | A | 10 March 2023 | None | | | |
| CN | 112789293 | A | 11 May 2021 | CA | 3110750 | A1 | 19 March 2020 |
| | | | | US | 2021277126 | A1 | 09 September 2021 |
| | | | | US | 2021275590 | A1 | 09 September 2021 |
| | | | | AU | 2019337759 | A1 | 11 March 2021 |
| | | | | EP | 3850013 | A1 | 21 July 2021 |
| | | | | EP | 3850013 | A4 | 05 October 2022 |
| | | | | TW | 202037607 | A | 16 October 2020 |
| | | | | WO | 2020052542 | A1 | 19 March 2020 |
| | | | | WO | 2020052543 | A1 | 19 March 2020 |
| | | | | EP | 3850011 | A1 | 21 July 2021 |
| | | | | EP | 3850011 | A4 | 19 October 2022 |
| | | | | TW | 202037606 | A | 16 October 2020 |
| | | | | CA | 3111458 | A1 | 19 March 2020 |
| | | | | AU | 2019340333 | A1 | 11 March 2021 |
| US | 2022354890 | A1 | 10 November 2022 | WO | 2022120942 | A1 | 16 June 2022 |
| | | | | EP | 4039712 | A1 | 10 August 2022 |
| | | | | GB | 202207722 | D0 | 13 July 2022 |
| | | | | GB | 2606869 | A | 23 November 2022 |
| | | | | JP | 2023510465 | A | 14 March 2023 |
| | | | | KR | 20220084040 | A | 21 June 2022 |
| | | | | AU | 2020480789 | A1 | 04 August 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ENGELS B**; **CAM H et al.** Retroviral Vectors for High-Level Transgene Expression in T Lymphocytes [J. *Human Gene Therapy*, 2003, vol. 14 (12), 1155-1168